(19) Europäisches Patentamt
European Patent Office
Office européen des brevets

(11) Publication number: 0 048 556
B1

(12) # EUROPEAN PATENT SPECIFICATION

(45) Date of publication of patent specification: 27.12.84

(51) Int. Cl.$^3$: A 61 L 15/06, C 09 J 3/02, A 61 F 5/44, C 09 J 3/14 // C08L35/08 ,(C08L35/08, 5/06, 5/00, 1/28)

(21) Application number: 81303942.7

(22) Date of filing: 27.08.81

(54) **Hydrocolloid containing paste-like composition.**

(30) Priority: 12.09.80 US 186791

(43) Date of publication of application: 31.03.82 Bulletin 82/13

(45) Publication of the grant of the patent: 27.12.84 Bulletin 84/52

(84) Designated Contracting States: AT BE CH DE FR GB IT LI LU NL SE

(56) References cited:
EP-A-0 018 093
FR-A-1 317 173
US-A-3 876 771

Römpps Chemie-Lexikon "Polyvinylpyrrolidone"

(73) Proprietor: E.R. Squibb & Sons, Inc.
Lawrenceville-Princeton Road
Princeton, N.J. 08540 (US)

(72) Inventor: Chen, James Ling
30 Fairview Avenue
East Brunswick New Jersey (US)
Inventor: Lavia, Anthony Laurence
66 Farms Road Circle
East Brunswick New Jersey (US)
Inventor: Riffkin, Charles
12 C Basswood Plaza
Cranbury New Jersey (US)

(74) Representative: Thomas, Roger Tamlyn et al
D. Young & Co. 10 Staple Inn
London WC1V 7RD (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention is directed to a composition of paste-like consistency useful for protecting and treating the skin contiguous to a stoma. The composition is alcohol based and includes as a film former the partial butyl ester of the polycarboxylic resin formed from vinyl methyl ether and maleic anhydride, one or more hydrocolloid gums, one or more gelling and thickening agents, a plasticizer, and other optional ingredients.

U.S. 3 876 771 claims in its broadest claim a gelatinous mixture for application to human skin, comprising:

25 to 90% by weight of isopropanol, 0.1 to 5% by weight of a preservative selected from the group consisting of sorbic acid, butyl parahydroxybenzoate and methyl parahydroxybenzoate, 0 to 50% by weight of polyvinyl pyrrolidone, 5 to 50% by weight of a film forming agent selected from the group consisting of monoisopropyl ester of polyvinyl methylether and polyvinyl methyl-ether, and 1.5 to 5% by weight of a gelling agent selected from the group consisting of hydroxypropyl cellulose and watersoluble polyacrylic acid crosslinked with 1% of polyallyl sucrose having an average of about 5.8 allyl groups for each sucrose molecule.

However, the disclosure of U.S. 3 876 771 indicates that polyvinyl pyrrolidone is present as a further film former and, except in the claim quoted, the maximum amount of polyvinyl pyrrolidone in any composition described is 5%.

The paste-like composition of this invention includes one or more water soluble or water swellable hydrocolloid substances. Suitable hydrocolloids include gelatin, pectin, sodium carboxymethylcellulose, guar gum, locust bean gum, karaya gum, and mixtures thereof. The hydrocolloid or mixture of hydrocolloid is present at from 20% to 40% by weight, preferably from 35% to 38% by weight, of the final composition. Preferred hydrocolloid mixtures contain gelatin, pectin, and sodium carboxymethylcellulose or these three hydrocolloids plus guar gum.

The composition includes as a film former the partial butyl ester of the polycarboxylic resin formed from vinyl methyl ether and maleic anhydride. Such resins conform generally to the structural formula

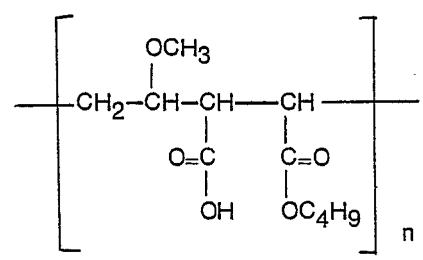

and are commercially available. This film forming resin is present at from 20% to 35% by weight, preferably from 25% to 28% by weight, of the final composition.

A plasticizer for the film forming resin is also included in the composition at from 0.1% to 5% by weight of the final composition. Suitable plasticizers include glyceryl triacetate, which is preferred, diethylene glycol, ethylene glycol monomethyl ether, dimethyl phthalate, diethyl phthalate, dimethyl sebacate, tricresyl phosphate, ethyl glycolate, etc.

A thickening or gelling agent is included in the composition at from 0.8% to 2% by weight of the final composition. The preferred gelling agent is glyceryl tris 12-hydroxystearate. Optionally, a small amount of up to 0.5% by weight of an auxiliary thickener such as hydroxypropyl cellulose can be included with the glyceryl tris 12-hydroxystearate.

Various optional ingredients can also be included in the final composition. For example an antioxidant such butylated hydroxyanisole or a deodorant or perfume agent or a coloring agent can be included in total at up to 5% by weight of the final composition. An organic acid such as alginic acid, which is preferred, citric, sebacic or malonic acid can be added at up to 5% by weight of the final composition to neutralize ammonia and other alkali intestinal fluid that is discharged from the stoma.

The various ingredients described above are combined into an alcohol based formulation of paste-like consistency. Ethanol is the preferred alcohol solvent though isopropanol could also be employed. The alcohol will be present at from 25% to 45% by weight of the final product.

The final composition may be prepared by first blending the hydrocolloids in finely divided powder form along with the gelling and thickening agents and any other optional ingredients. This dry blend is then added with stirring to an alcoholic solution of the film former resin and plasticizer. Additional alcohol may be added as needed to adjust the viscosity of the final product to the desired paste-like consistency. The composition may be packaged in conventional squeeze tube containers.

The following examples are illustrative of the invention. Other suitable compositions can be obtained by minor variations in the amounts of ingredients employed.

Example 1

A paste-like composition is prepared containing on a weight percent basis:

| | |
|---|---|
| Partial butyl ester of the polycarboxylic resin formed from vinyl methyl ether and maleic anhydride (Gantrez ES-425, GAF) | 26% |
| Glyceryl triacetate | 0.5% |
| Pectin | 12.5% |
| Gelatin | 12.5% |
| Sodium carboxymethylcellulose | 12.5% |
| Glyceryl tris 12-hydroxy stearate (Thixcin E, NL Corp.) | 1% |
| Ethanol (U.S.P. 95%) | 35% |

Gantrez and Thixcin are Trade Marks

A 100 gm. batch of the above composition is prepared as follows:

1 g of Thixcin E is added to 12.5 gm. of pectin and dry blended. 12.5 gm. of gelatin of 12.5 gm of sodium carboxymethylcellulose are then added and stirring is continued to produce a uniform blend. This blend is added to a solution of 52 gm. of Gantrez ES-425, which is a 1:1 mixture on a weight basis of the resin and ethanol, and 0.5 gm. of glyceryl triacetate. Stirring is continued and an additional 9 gm. of ethanol are added to yield the desired paste-like composition.

Example 2

Following the procedure of Example 1, a paste-like composition is prepared containing on a weight percent basis:

| | |
|---|---|
| Partial butyl ester of the polycarboxylic resin formed from vinyl methyl ether and maleic anhydride (Gantrez ES-425, GAF) | 26% |
| Glyceryl triacetate | 0.5% |
| Pectin | 5% |
| Gelatin | 5% |
| Sodium carboxymethyl-cellulose | 5% |
| Guar gum | 20% |
| Alginic acid | 1.5% |
| Glyceryl tris 12-hydroxy-stearate (Thixin E, NL Corp.) | 1.5% |
| Ethanol (U.S.P. 95%) | 35.5% |

Examples 3—10

Following the procedure of Example 1, additional compositions can be prepared as follows (the ingredients are listed on weight percent basis):

| Example | 3 | 4 | 5 | 6 | 7 | 8 | 9 | 10 |
|---|---|---|---|---|---|---|---|---|
| Ingredient | | | | | | | | |
| Gantrez ES–425 | 20% | 35% | 25% | 25% | 28% | 30% | 30% | 35% |
| Glyceryl triacetate | 0.3% | 3% | 0.5% | 0.5% | 0.5% | 1% | 0.5% | 2% |
| Pectin | 10% | – | – | 12.5% | 6% | 15% | 10% | 7% |
| Gelatin | 10% | – | 5% | 12.5% | 6% | – | 10% | 7% |
| Sodium CMC | 10% | – | 5% | 12.5% | 6% | – | 10% | 7% |
| Guar gum | 10% | 20% | 20% | – | 20% | 20% | 10% | – |
| Karaya gum | – | – | 5% | – | – | – | – | – |
| Alginic acid | 1% | 1% | 1.5% | 1.5% | – | – | – | – |
| Thixcin E | 1% | 1% | 1.5% | 1% | 1% | 1% | 1% | 1% |
| Klucel (hydroxypropyl cellulose) | 0.2% | – | – | – | 0.2% | – | – | – |
| Ethanol (U.S.P., 95%) | 37.5% | 40% | 36.5% | 34.5% | 32.3% | 33% | 28.5% | 41% |

## Claims

1. A composition for use in treating or protecting human skin comprising:

a) from 20% to 40% by weight of one or more water soluble or water swellable hydrocolloids selected from pectin, gelatin, sodium carboxymethylcellulose, guar gum, karaya gum, and locust bean gum;

b) from 20% to 35% by weight of a film former which is the partial butyl ester of the polycarboxylic resin formed from vinyl methyl ether and maleic anhydride;

c) from 0.1% to 5% by weight of a plasticizer for said film former;

d) from 0.8% to 2% by weight of gelling and thickening agents;

e) from 25% to 45% by weight of alcohol.

2. The composition of Claim 1 wherein the alcohol is ethanol, the plasticizer is glyceryl triacetate, and the gelling agent is glyceryl tris 12-hydroxystearate.

3. The composition of Claim 2 wherein said hydrocolloid or mixture of hydrocolloids is present at from 35% to 38% by weight of the composition and said film former is present at from 25% to 28% by weight of the composition.

4. The composition of Claim 3 wherein said hydrocolloids are a mixture of pectin, gelatin, and sodium carboxymethylcellulose.

5. The composition of Claim 4 containing about 12.5% by weight of pectin, about 12.5% by weight of gelatin, and about 12.5% by weight of sodium carboxymethylcellulose.

6. The composition of Claim 3 wherein said hydrocolloids are a mixture of pectin, gelatin, sodium carboxymethylcellulose, and guar gum.

7. The composition of Claim 6 containing about 5% by weight of pectin, about 5% by weight of gelatin, and about 5% by weight of sodium carboxymethylcellulose, and about 20% by weight of guar gum.

8. The composition of Claim 3 including up to 5% by weight of an organic acid selected from alginic acid, citric acid, malonic acid, and sebacic acid.

9. The composition of Claim 8 including about 1% by weight of alginic acid.

10. The composition of Claim 2 including up to 0.5% by weight of hydroxypropyl cellulose as an auxiliary thickening agent.

11. A process for making the composition of any preceding claim, which comprises blending component (a) in finely divided powder form with component (d) and adding the blend with stirring to a solution of component (b) and component (c) in component (e).

## Revendications

1. Composition pouvant être utilisée pour le traitement ou la protection de la peau humaine, comprenant:

a) de 20% à 40% en poids d'un ou plusieurs hydrocolloïdes solubles dans l'eau ou gonflant dans l'eau qui sont choisis entre la pectine, la gélatine, la carboxyméthylcellulose sodique, le guar, le karaya et la gomme de caroubier;

b) de 20% à 35% en poids d'un feuillogène qui est l'ester butylique partiel de la résine polycarboxylique formée à partir du vinyl méthyl éther et de l'anhydride maléique;

c) de 0,1% à 5% en poids d'un plastifiant pour ledit feuillogène;

d) de 0,8% à 2% en poids d'agents gélifiants et épaississants;

e) de 25% à 45% en poids d'alcool.

2. Composition selon la revendication 1, dans laquelle l'alcool est l'éthanol, le plastifiant est le triacétate de glycéryle, et l'agent gélifiant est le tris 12-hydroxystéarate de glycéryle.

3. Composition selon la revendication 2, dans laquelle ledit hydrocolloïde ou ledit mélange d'hydrocolloïdes représente de 35% à 38% du poids de la composition, et ledit feuillogène représente de 25% à 28% du poids de la composition.

4. Composition selon la revendication 3, dans laquelle lesdits hydrocolloïdes sont un mélange de pectine, de gélatine et de carboxyméthylcellulose sodique.

5. Composition selon la revendication 4, contenant environ 12,5% en poids de pectine, environ 12,5% en poids de gélatine, et environ 12,5% en poids de carboxyméthylcellulose sodique.

6. Composition selon la revendication 3, dans laquelle lesdits hydrocolloïdes sont un mélange de pectine, de gélatine, de carboxyméthylcellulose sodique et de guar.

7. Composition selon la revendication 6, contenant environ 5% en poids de pectine, environ 5% en poids de gélatine, environ 5% en poids de carboxyméthylcellulose sodique, et environ 20% en poids de guar.

8. Composition selon la revendication 3, contenant jusqu'à 5% en poids d'un acide organique choisi entre l'acide alginique, l'acide citrique, l'acide malonique et l'acide sébacique.

9. Composition selon la revendication 8, contenant environ 1% en poids d'acide alginique.

10. Composition selon la revendication 2, contenant jusqu'à 0,5% en poids d'hydroxypropyl cellulose à titre d'agent épaississant auxiliaire.

11. Procédé de préparation de la composition selon l'une quelconque des revendications précédentes, qui consiste à mélanger le composant (a), sous la forme d'une poudre finement divisée, avec le composant (d), et à ajouter le mélange, en agitant, à une solution du composant (b) et du composant (c) dans le composant (e).

**Patentansprüche**

1. Eine Zusammensetzung zur Behandlung oder zum Schutz von menschlicher Haut enthaltend:

(a) 20 bis 40 Gewichtsprozent eines oder mehrerer wasserlöslicher oder in Wasser aufquellender Hydrokolloide ausgewählt aus Pectin, Gelatine, Natriumcarboxymethylcellulose, Guar Gum, Karaya-Gummi und Johannisbrotkerngummi;

b) 20 bis 35 Gewichtsprozent eines Filmbildners, der ein teilweise mit Butylalkohol verestertes Polycarboxykunstharz aus Vinylmethyläther und Maleinsäureanhydrid darstellt;

c) 0,1 bis 5 Gewichtsprozent eines Weichmachers für sanften Filmbildner,

d) 0,8 bis 2 Gewichtsprozent eines Mittels zur Gelbildung bzw. eines Dickmittels.

e) 25 bis 45 Gewichtsprozent Alkohol.

2. Dis Zusammensetzung nach Anspruch 1, in der der Alkohol Äthanol ist, der Weichmacher Glyceryltriacetat ist und das Mittel zur Gelbildung Glyceryul-tris-12-hydroxystearat ist.

3. Die Zusammensetzung nach Anspruch 2, in der besagtes Hydrokolloid oder ein Gemisch von Hydrokolloiden zu 35 bis 38 Gewichtsprozent in der Zusammensetzung vorliegt und besagter Filmbildner zu 25 bis 28 Gewichtsprozent in der Zusammensetzung vorliegt.

4. Die Zusammensetzung nach Anspruch 3, in der die besagten Hydrokolloide ein Gemisch aus Pectin, Gelatine und Natriumcarboxymethylcellulose darstellen.

5. Die Zusammensetzung nach Anspruch 4, enthaltend etwa 12,5 Gewichtsprozent Pectin, etwa 12,5 Gewichtsprozent Gelatine und etwa 12,5 Gewichtsprozent Natriumcarboxymethylcellulose.

6. Die Zusammensetzung nach Anspruch 3, in der besagte Hydrokolloide ein Gemisch von Pectin, Gelatine, Natriumcarboxymethylcellulose und Guar Gum darstellen.

7. Die Zusammensetzung nach Anspruch 6, enthaltend etwa 5 Gewichtsprozent Pectin, etwa 5 Gewichtsprozent Gelatine und etwa 5 Gewichtsprozent Natriumcarboxymethylcellulose und etwa 20 Gewichtsprozent Guar Gum.

8. Die Zusammensetzung nach Anspruch 3 einschließlich bis zu 5 Gewichtsprozent einer organischen Säure, ausgewählt aus Alginsäure, Citronensäure, Malonsäure und Sebacinsäure.

9. Die Zusammensetzung nach Anspruch 8 einschließlich etwa 1 Gewichtsprozent Alginsäure.

10. Die Zusammensetzung nach Anspruch 2 einschließlich bis zu 0,5 Gewichtsprozen Hydroxypropylcellulose als Hilfsdickmittel.

11. Ein Verfahren zur Herstellung der Zusammensetzung nach einem der vorstehenden Ansprüche, gekennzeichnet durch Vermischen der Komponente (a) in feinverteilter Pulverform mit der Komponente (d) und Zugabe dieser Mischung unter Rühren zu einer Lösung der Komponente (b) und der Komponente (c) in der Komponente (e).